# EUROPÄISCHE PATENTSCHRIFT

(11) **EP 0 920 879 B1**
(45) Veröffentlichungstag und Bekanntmachung des Hinweises auf die Patenterteilung: **23.04.2003**
(21) Anmeldenummer: 98121812.6
(22) Anmeldetag: 17.11.1998
(51) Int. Cl.: A61M 5/00, A61M 5/31, A61M 5/315, A61L 29/00

(54) **Pharmapackmittel, bestehend aus einem länglichen Kunststoff-Hohlkörper und Verfahren zu seiner Herstellung**
Pharmaceutical packaging means having an elongated hollow body of plastics, and manufacturing process therefor
Moyen d'emballage pour produits pharmaceutiques constitué d'un corps creux oblong en plastic et son procédé de fabrication

(30) Priorität: 04.12.1997 DE 19753766
(43) Veröffentlichungstag der Anmeldung: 09.06.1999
(73) Patentinhaber: Schott Glas, 55122 Mainz (DE); Carl-Zeiss-Stiftung trading as Schott Glas, 55122 Mainz (DE)
(72) Erfinder: Heinz, Jochen, Dr., 55578 Vendersheim (DE); Weber, Gerhard, 55234 Bechenheim (DE); Spallek, Michael, Dr., 55218 Ingelheim (DE)
(74) Vertreter: Fuchs Mehler Weiss & Fritzsche

(56) Entgegenhaltungen:
- EP-A- 0 227 340
- EP-A- 0 709 105
- EP-A- 0 756 901
- US-A- 4 509 981
- US-A- 5 338 312

## Beschreibung

Die Erfindung betrifft ein Pharmapackmittel, bestehend aus einem länglichen Kunststoff-Hohlkörper, der zumindest über einen Teil seiner axialen Länge zylindrisch ausgebildet, und in diesem zylindrischen Bereich mit einer Silikon-Gleitschicht versehen ist, sowie der durch dicht sitzende Stopfen verschließbar ist.

Die Erfindung betrifft ferner ein Verfahren zu seiner Herstellung.

Bei Pharmapackmitteln, bestehend aus länglichen Kunststoff-Hohlkörpern ist es notwendig, daß ein in den Hohlraum eingebrachter elastomerer Verschluß, z.B. ein Kolben bzw. ein Kolbenstopfen, geschmeidig an der Innenwandung des Hohl-Körpers gleitet. Typische Beispiele sind Kunststoff-Spritzenzylinder für vorfüllbare Spritzen, Spritzenkörper von Zylinderampullen und Karpulen, aber auch Kolbenbüretten in der chemischen Analytik.

Um diesem Problem gerecht zu werden, wird typischerweise auf die Innenwandung des Kunststoff-Hohlkörpers eine Silikon-Gleitschicht aufgebracht.

Gemäß der US-A-4,767,414 und der EP 0201 915 B1 wird Silikonöl auf die Oberfläche der Innenwandung aufgetragen, wobei zur Erzielung einer guten Haftung die Oberfläche mit einem ionisieren Plasma aktiviert wird und das aufgetragene Silikonöl ebenfalls mit diesem ionisieren Plasma behandelt wird.

Dieses Verfahren ist wegen der notwendigen Mehrfachschritte relativ umständlich. Zudem enthält das Silikonöl verhältnismäßig viel nicht reaktives, d.h. freies Silikonöl, das mit dem Inhalt des Kunststoff-Hohlkörpers beim Betätigen des Kolbens austreten kann. Das Einbringen von Silikonöl in den menschlichen Körper im Fall von Spritzen sollte jedoch aus den inzwischen bekannten Gründen auf ein Minimum reduziert werden.

Die US-A-4,844,986 beschreibt ein Verfahren zum Aufbringen einer Silikon-Gleitschicht auf die Innenwandung eines Kunststoff-Hohlkörpers mittels einer Plasmapolymerisation zur Vorbereitung einer Beschichtung mit zugeführtem Polydimethylsiloxan.

Auch dieses Verfahren benötigt eine Aktivierung der Kunststoffoberfläche und ein mehrstufiges Auftragsverfahren.

Die EP 0 329 041 beschreibt ein Verfahren zur Beschichtung der Innenwand eines Kunststoff-Hohlkörpers mit einem Polysiloxan, oder Polysilan oder Polysilazan mittels eines ionisierenden Plasmas, wobei zunächst eine Haftschicht aus diesem Material erzeugt und darauf eine dünne Gleitschicht aufgetragen wird.

Auch dieses Verfahren benötigt somit eine Aktivierung der Kunststoffoberfläche und besteht aus mehreren Stufen.

Gleiches gilt für das Verfahren nach der EP 0 302 625 B1, gemäß dem zunächst die Oberfläche der Innenwandung des Kunststoff-Hohlkörpers mit einem Plasma behandelt wird, um eine plasmabehandelte Polymeroberfläche zu erhalten, auf der ohne Plasmabehandlung eine Schicht eines Polysiloxan-Gleitmittels aufgebracht wird, das die plasmabehandelte Oberfläche durch Vorgabe einer entsprechenden Oberflächenspannung vollständig benetzt.

Die US-A-5,338,312 schließlich beschreibt eine Beschichtung der Innenwandung eines Kunststoff-Hohlkörpers, bei der zwei Gleitschichten übereinander angebracht sind, nämlich eine auf der Innenwand aufgebrachte Grundschicht aus Silikonöl mit hoher Viskosität in Verbindung mit einer Haftscicht und einer Oberflächenschicht aus Silikonöl mit niedriger Viskosität, wobei die Silikonöle in den einzelnen Schichten jeweils vorzugsweise durch ein ionisierendes Plasma vernetzt werden.

Zum Aufbringen von Gleitschichten nach diesem Verfahren ist ebenfalls eine aufwendige Verfahrenstechnik notwendig. Zum anderen enthält die Oberflächenschicht viel freies, d.h. nicht reaktives Silikonöl.

Der Erfindung liegt die Aufgabe zugrunde, das eingangs bezeichnete Pharmapackmittel, bestehend aus einem länglichen Kunststoff-Hohlkörper, der zumindest über einen Teil seiner axialen Länge zylindrisch ausgebildet und in diesem zylindrischen Bereich mit einer Silikon-Gleitschicht versehen ist, sowie der durch dicht sitzende Stopfen verschließbar ist, so auszubilden sowie das Verfahren zu seiner Herstellung so zu führen, daß es auf einfache Weise in einem einstufigen Verfahren herstellbar ist, und daß die Kontamination des Stoffes, mit dem ggf. der Kunststoff-Hohlkörper befüllt wird, mit Silikontröpfchen minimiert ist.

Die Lösung dieser Aufgabe gelingt erfindungsgemäß dadurch, daß die Gleitschicht aus einer Mischung von reaktivem Silikonöl mit nichtreaktivem Silikonöl besteht, wobei der Anteil des nicht reaktiven Silikonöles in der Mischung im Bereich von 5% bis 85% liegt und die Viskosität des reaktiven Silikonöles bei Einkomponenten-Systemen im Bereich von 10 000 bis 100 000 centistokes, bei Mehrkomponenten-Systemen im Bereich von 200 bis 5 000 centistokes, und die Viskosität des nichtreaktiven Silikonöles im Bereich von 350 bis 20 000 centistokes liegt.

Hinsichtlich des Verfahrens zur Herstellung des Pharmapackmittels, bestehend aus einem länglichen Kunststoff-Hohlkörper, gelingt die Lösung der Aufgabe erfindungsgemäß mit den Schritten:
- Ausformen des Kunststoff-Hohlkörpers,
- Bereitstellen einer Mischung aus reaktivem Silikonöl mit nichtreaktivem Silikonöl,
- Auftragen der Mischung der Silikonöle auf die Innenwandung des Kunststoff-Hohlkörpers im zylindrischen Bereich,
- Vernetzen der gemischten Silikonöle in der aufgetragenen Schicht, und
- Weiterverarbeitung des so mit einer Gleitschicht versehenen Kunststoff-Hohlkörpers entsprechend seiner vorgesehenen Verwendung.

Das erfindungsgemäße Prinzip besteht somit in der Verwendung einer Mischung von reaktivem Silikonöl und nicht reaktivem Silikonöl. Es hat sich überraschenderweise gezeigt, daß bei der Verwendung einer solchen Mischung es nicht notwendig ist, die Oberfläche des Kunststoff-Hohlkörpers zu aktivieren.

Dies trifft gemäß einer Weiterbildung der Erfindung besonders dann zu, wenn das Kunststoffmaterial, aus dem der Kunststoff-Hohlkörper hergestellt ist, ein Cycloolefinpolymer oder ein Copolymer davon (COC) ist, und das Auftragen der Mischung der Silikonöle unmittelbar nach dem Ausformen des Kunststoff-Hohlkörpers auf seine noch warme, eine Temperatur vorzugsweise über 40° C aufweisende Innenwandung erfolgt.

Die Erfindung ermöglicht es daher, die Silikon-Gleitschicht des Kunststoff-Hohlkörpers in einem einstufigen Verfahren, d.h. ohne aufwendige Aktivierung der Oberfläche bzw. mehrstufige Auftragsverfahren aufzubringen. Das anteilige reaktive Silikonöl bildet nach der Vernetzung praktisch die haftende Grundschicht, in die das anteilige nicht reaktive Silikonöl lokal eingebettet ist und die Gleitfähigkeit bewirkt.

Reaktive Silikonöle enthalten im Gegensatz zu nicht reaktiven Silikonölen reaktive funktionelle Gruppen, die eine Vernetzung in sich und ggf. auch mit anderen Oberflächen ermöglichen.

Es bildet sich daher eine Trenn- und Gleitschicht zwischen Behälter (Kunststoff-Hohlkörper) und beweglicher Elastomerkomponente (Stopfen) aus.

Der Aufbau ist gemäß seiner Funktion ein (Siloxan)-Molekül, welches je nach Aushärtungsmechanismus funktionelle Gruppen enthält.

Ein reaktives Silikonöl enthält insbesondere Vinylgruppen, die unter dem Einfluß von Temperatur und UV-Bestrahlung vernetzen: Die Vernetzungsreaktion wird daher im speziellen von Platinverbindungen katalysiert.

Das ausgehärtete Silikonöl bildet dann einen festen Film, der als Trennschicht zwischen Kunststoff-Hohlkörper und Kolbenstopfen fungiert. Ein Festbacken des insbesondere aus Brombutyl bestehenden Stopfens an der COC-Oberfläche des Kunststoff-Hohlkörpers wird dadurch verhindert. Zur Herabsetzung der Gleitreibung wird dem reaktiven Silikonöl Dimethypoly-Siloxane als nicht reaktives Silikonöl beigemischt.

Ein Silikonierungsmittel auf Basis eines reaktiven Silikonöls setzt sich daher vorzugsweise zusammen aus einem vinylgruppenhaltigen Silikonöl, Vernetzer, Katalysator, Lösungsmittel und nicht reaktive Dimethypolysiloxane der Viskositätsstufen 350-20000cSt.

Die Verwendung einer Mischung aus reaktiven und nicht reaktiven Silikonölen als Gleitmittel ist an sich durch die US-4,806,430 und US-4,720,521 bekannt geworden. In den bekannten Fällen werden jedoch ausschließlich metallische Substrate, beispielsweise Injektionsnadeln, Rasierklingen oder dergleichen beschichtet, wogegen es im Fall der Erfindung um das Beschichten von Kunststoffoberflächen eines Pharmapackmittels geht. Für diese Problemstellung gibt der vorgenannte Stand der Technik dem Fachmann keine Anregungen. Er hat daher die Entwicklung auf dem Gebiet der Beschichtung von Kunststoffen nicht beeinflussen können.

Da die Substrate gänzlich verschieden sind, müssen es auch die entsprechenden Beschichtungsverfahren sein, insbesondere weil die Haftungseigenschaften entsprechend unterschiedlich sind. Bekannt ist dies z.B. auch bei der Lackierung von Autos, wo die Lackierung der metallischen Karosserie nicht in gleicher Weise bei der Lackierung der Kunststoffstoßstangen, die typischerweise aus Polyolefin bestehen, angewendet werden kann. Der erfindungsgemäße Kunststoffbehälter besteht ferner aus einem diffusionsoffenen Kunststoff, so daß Komponenten der Silikonisierung in den Kunststoffkörper einwandern könnten und somit die Gleitfähigkeit nicht mehr gegeben wäre. Diese Problemstellung gibt es bei den diffusionsdichten Metall-Substraten nach dem vorgenannten Stand der Technik prinzipiell nicht. Bei Pharmaartikeln werden auch die Kunststoff-Hohlkörper typischerweise durch dicht sitzende Elastomer-Stopfen verschlossen. Durch die dabei notwendige Dichtkraft kann Silikon verdrängt werden oder ebenfalls durch Diffusion in den Stopfen aufgenommen werden, so daß ein Gleiten des Stopfens nach längerer Lagerung des Pharmapackmittels nicht mehr möglich wäre.

Als Bestandteil des Kunststoff-Hohlkörpers ist schließlich die Silikonisierung während der gesamten Lagerzeit des befüllten Pharmapackmittels (bis zu 5 Jahre) einem Flüssigkeitskontakt ausgesetzt, was bei den Produkten nach dem vorgenannten Stand der Technik in keiner Weise der Fall ist.

Im Grundsatz ist der zylindrische Bereich des Kunststoff-Hohlkörpers über seine volle axiale Länge mit der Silikon-Gleitschicht versehen.

Besondere Vorteile werden jedoch erreicht, wenn das Auftragen der Mischung der Silikonöle gezielt in Bezug auf die axiale Ausdehnung des zylindrischen Bereiches erfolgt, d.h. wenn gezielt Bereiche von der Beschichtung ausgenommen sind. Durch dieses selektive teilweise Beschichten können weitere Funktionen erreicht werden, wie das Beispiel der selektiven Beschichtung eines Spritzenzylinders, in dem ein Gurummikolben verschiebbar gleitet, zeigt. Durch Nichtbeschichten des hinteren Endes des Spritzenzylinders auf einer Länge von ca. 1 cm, läßt es sich so erreichen, daß selbst ein mit einem Gleitmittel versehener Gummikolben nicht aus dem rückwärtigen Ende des Spritzenzylinders herausgezogen werden kann. Diese selektive Beschichtung ermöglicht es somit, ohne großen Aufwand eine Kolbenbremse zu realisieren.

Andererseits ist es auch möglich, am kopfseitigen Ende des Spritzenzylinders, ebenfalls eine Breite von ca. 1 cm ohne Gleitschicht-Beschichtung zu belassen. Damit wird der umgekehrte Effekt erreicht: dem Gummikolben wird auf dieser Strecke ein erhöhter Reibungswiderstand entgegengesetzt, der Kolben läßt sich jedoch aufgrund der vorangeschobenen Gleitmittelschicht noch bis ans vordere Ende des Spritzenzylinders bewegen. Durch den Verbrauch der Gleitmittelschicht in diesem Bereich, ist es jedoch dann nicht mehr möglich, den Kolben wieder zurück zu bewegen. Somit wird in einfachster Weise die Funktion einer nicht wiederverwendbaren Spritze realisiert.

Durch die Erfindung läßt sich ferner mit Vorteil das sehr zähe reaktive Einkomponenten-Silikon in einer Mischung mit niedrig viskosem, nicht reaktivem Silikonöl wesentlich leichter verarbeiten und aufbringen, da letzteres wie ein Verdünnungsmittel wirkt.

Gegenüber dem Stand der Technik ergibt sich weiterhin neben deutlichen verfahrenstechnischen Vorteilen und funktionalen Erweiterungen auch eine deutlich geringere Kontamination des Inhalts, beispielsweise einer vorgefüllten Einmalspritze durch Silikontröpfchen, da erfindungsgemäß der Gehalt an freiem, d.h. nicht reaktivem Silikon in der Gleitschicht auf ein Minimum gesenkt ist.

Weitere Vorteile des erfindungsgemäßen Kunststoff-Hohlkörpers sind:
- Der Unterschied zwischen Haftreibung und Gleitreibung ist beim erfindungsgemäßen Kunststoff-Hohlkörper bei weitem nicht so ausgeprägt wie bei den bekannten Hohlkörpern. Dadurch wird vermieden, daß durch verhältnismäßig hohe Haftreibungskräfte Druckkräfte notwendig sind, die beispielsweise zu einem ruckartigen Austreten des Inhaltes aus einem Spritzenkörper führen können.
- Es wird eine verbesserte Haftung der Gleitschicht auf der Oberfläche der Innenwandung erzielt.
- Es ist möglich, die sonst übliche Silikonisierung von Gummikolben, die in dem zylindrischen Bereich verschiebbar eingebracht werden, auf ein Minimum zu reduzieren.

Es hat sich gezeigt, daß auch die erfindungsgemäß zusammengesetzte Gleitschicht mit Standardmethoden sterilisierbar ist (typischerweise Autoklavierung in Wasserdampf 121° C/20 min.), und dabei wenig Silikonpartikel an den Inhalt abgegeben werden. Diese Eigenschaften weisen sowohl vor, als auch nach Bestrahlung des mit der Gleitschicht versehenen Körpers durch energiereiche Strahlung (γ-Strahlung, Elektronenstrahl, energiereiche Lichtblitze, etc.) keine einschränkenden Änderungen auf.

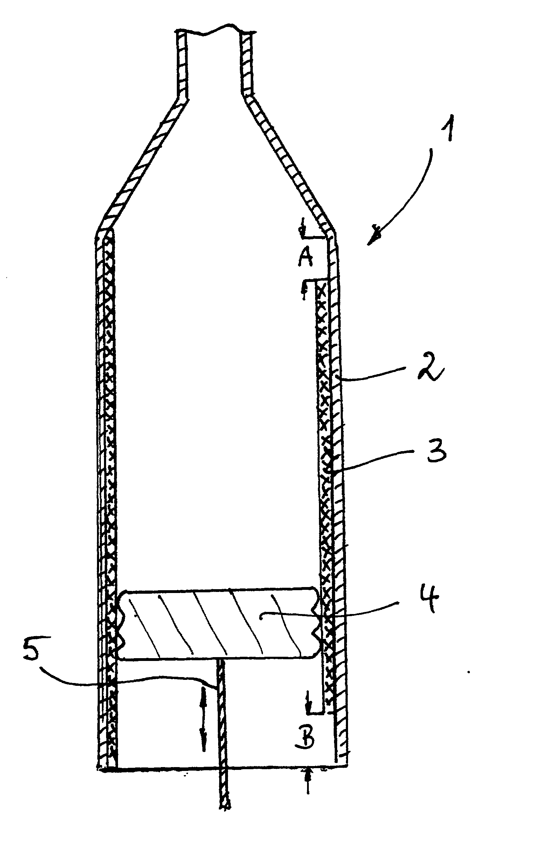

Weitere ausgestaltende Merkmale der Erfindung ergeben sich anhand eines in der Zeichnung dargestellten Ausführungsbeispieles. In dem Ausführungsbeispiel ist in einem Längsschnitt ein Spritzenkörper 1 für eine vorfüllbare Spritze als Beispiel eines länglichen Kunststoff-Hohlkörpers dargestellt. Es versteht sich, daß, wie eingangs erwähnt, der längliche Kunststoff-Hohlkörper auch ein Spritzenkörper für Zylinderampullen, Karpulen, oder eine Kolbenbürette und dergleichen sein kann.

Die Wandung 2 des Spritzenkörpers 1 ist innen mit einer Gleitschicht 3 versehen.

Die Darstellung in der einzigen Figur der Zeichnung ist dabei nicht maßstäblich, um die aufgebrachte Silikon-Gleitschicht 3 darstellen zu können.

Diese Silikon-Gleitschicht 3 ist im zylindrischen Teil des Spritzenkörpers 1 aufgebracht. Sie kann sich dabei, wie im linken Teil der Figur dargestellt, über die gesamte Länge dieses zylindrischen Bereiches erstrecken. Es können jedoch auch, wie im rechten Teil der Figur dargestellt, die Endabschnitte A und B unbeschichtet bleiben.

Innerhalb des Spritzenzylinders ist ein Kolben 4 positioniert, der mittels einer lösbar angebrachten Kolbenstange 5 verschiebbar ist. Die Silikon-Gleitschicht 3 sorgt dabei für eine geschmeidige Gleitbewegung des Kolbens 4. Der gleitmittelfreie Endabschnitt B wirkt als Kolbenbremse, d.h. verhindert, daß der Kolben 4 aus dem Spritzenzylinder herausgezogen werden kann. Der freie Endabschnitt A sorgt dafür, daß sich nach dem Applizieren, d.h. wenn der Kolben 4 die kopfseitige Position erreicht hat, der Kolben praktisch nicht mehr zurückbewegen läßt, wodurch eine systemwidrige Wiederverwendung einer vorgefüllten Einmalspritze verhindert wird. Erfindungsgemäß besteht die Silikon-Gleitschicht 3 aus einer Mischung von reaktivem Silikonöl hoher Viskosität mit nichtreaktivem Silikonöl niedriger Viskosität.

Die Viskosität des reaktiven Silikonöles liegt bei Einkomponenten-Systemen im Bereich von 10 000 bis 100 000 centistokes, bei Mehrkomponenten-Systemen im Bereich von 200 bis 5 000 centistokes, wogegen die Viskosität des nicht reaktiven Silikonöles im Bereich von 350 bis 20 000 centistokes liegt.

Zum Auftragen der Silikonöle können diese noch verdünnt werden, insbesondere wenn das Auftragen durch Sprühen erfolgt.

Die Dicke der Silikon-Gleitschicht 3 liegt vorzugsweise im Bereich von 10 nm bis 1 µm.

Der Anteil des freien, d.h. nicht reaktiven Silikonöles hängt ab von dem Lösungsmittelanteil und der Viskosität des verwendeten reaktiven Silikonöls. Er liegt im Bereich von 5% bis 85% bezogen auf die Mischung.

Die Silikonöle können aus den nachstehenden Silikonverbindungen bestehen:
Nicht vernetzte, polare Silikone oder Polysiloxane, insbesondere nicht reaktive Polydimethyldisiloxane (PDMS) oder reaktive Polymersiloxane, Copolymere von Alkylamin-modifizierten Methoxysiloxane, Polysiloxane mit einer Aminoalkyl-Gruppe.

Das Vernetzen erfolgt mit bekannten Methoden, insbesondere mit UV-Licht oder durch Plasmapolymerisation.

Das Kunststoffmaterial, aus dem der Kunststoff-Hohlkörper 1 jeweils hergestellt ist, bestimmt sich nach den speziellen Forderungen im konkreten Anwendungsfall. Bei Anwendungen, bei denen es auf eine gute Maßhaltigkeit ankommt, wie beispielsweise bei Spritzenkörpern und Bürettenzylindern für Kolbenbüretten, findet vorzugsweise ein relativ formstabiler Kunststoff Anwendung, vorzugsweise ein Cycloolefincopolymer (COC), der im Spritzgießverfahren ausgeformt wird. Vorzugsweise wird dabei ein in-line-Verfahren angewendet, bei dem in einer Fertigungslinie der Kunststoff-Hohlkörper ausgeformt, befüllt und verschlossen wird. Ein derartiges Verfahren ist Gegenstand der älteren deutschen Patentanmeldung 196 52 708.2-35. Bei Zugrundelegung dieses Verfahrens ist es möglich, die Mischung der Silikonöle unmittelbar nach dem Ausformen des Kunststoff-Hohlkörpers auf dessen noch warme, eine Temperatur von über 40° C aufweisende Innenwandung aufzubringen, was eine besonders gute Haftung der Silikon-Gleitschicht bewirkt, insbesondere dann, wenn der Kunststoff-Hohlkörper aus einem COC-Kunststoff hergestellt ist.

Das Auftragen der Silikonöl-Mischung erfolgt mit bekannten Methoden, insbesondere durch Aufsprühen oder Aufreiben.

## Patentansprüche

1. Pharmapackmittel, bestehend aus einem länglichen Kunststoff-Hohlkörper (1), der zumindest über einen Teil seiner axialen Länge zylindrisch ausgebildet und in diesem zylindrischen Bereich mit einer Silikon-Gleitschicht (3) versehen ist, sowie der durch dicht sitzende Stopfen (14) verschließbar ist, **dadurch gekennzeichnet, daß** die Gleitschicht (3) aus einer Mischung von reaktivem Silikonöl mit nicht reaktivem Silikonöl besteht, wobei der Anteil des nicht reaktivem Silikonöles in der Mischung im Bereich von 5% bis 85% liegt und die Viskosität des reaktiven Silikonöles bei Einkomponenten-Systemen im Bereich von 10 000 bis 100 000 centistokes, bei Mehrkomponenten-Systemen im Bereich von 200 bis 5 000 centistokes, und die Viskosität des nichtreaktiven Silikonöles im Bereich von 350 bis 20 000 centistokes liegt.

2. Pharmapackmittel nach Anspruch 1, **dadurch gekennzeichnet, daß** die Dicke der Gleitschicht (3) im Bereich von 10 nm bis 1 µm liegt.

3. Pharmapackmittel nach Anspruch 1 oder 2, **dadurch gekennzeichnet, daß** der zylindrische Bereich des Kunststoff-Hohlkörpers (1) über seine volle axiale Länge mit der Gleitschicht (3) versehen ist.

4. Pharmapackmittel nach einem der Ansprüche 1 bis 3, **dadurch gekennzeichnet, daß** einer der Endabschnitte des zylindrischen Bereiches des Kunststoff-Hohlkörpers (1) unbeschichtet ist.

5. Pharmapackmittel nach einem der Ansprüche 1 bis 3, **dadurch gekennzeichnet, daß** beide Endabschnitte (A, B) des zylindrischen Bereiches des Kunststoff-Hohlkörpers (1) unbeschichtet sind.

6. Pharmapackmittel nach einem der Ansprüche 1 bis 5, **dadurch gekennzeichnet, daß** das Kunststoffmaterial, aus dem der Kunststoff-Hohlkörper (1) hergestellt ist, ein Cycloolefinpolymer oder ein Copolymer davon ist.

7. Verfahren zur Herstellung des Pharmapackmittels, bestehend aus einem länglichen Kunststoff-Hohlkörper, nach einem der Ansprüche 1 bis 6 mit den Schritten:
- Ausformen des Kunststoff-Hohlkörpers,
- Bereitstellen einer Mischung aus reaktivem Silikonöl mit nicht reaktivem Silikonöl,
- Auftragen der Mischung der Silikonöle auf die Innenwandung des Kunststoff-Hohlkörpers im zylindrischen Bereich,
- Vernetzen der gemischten Silikonöle in der aufgetragenen Schicht, und
- Weiterverarbeitung des so mit einer Gleitschicht versehenen Kunststoff-Hohlkörpers entsprechend seiner vorgesehenen Verwendung.

8. Verfahren nach Anspruch 7, **dadurch gekennzeichnet, daß** das Auftragen der Mischung der Silikonöle gezielt in Bezug auf die axiale Ausdehnung des zylindrischen Bereiches erfolgt.

9. Verfahren nach Anspruch 8, **dadurch gekennzeichnet, daß** durch ein gezieltes selektives Auftragen der Mischung der Silikonöle ein Endabschnitt des zylindrischen Bereiches unbeschichtet bleibt.

10. Verfahren nach Anspruch 8, **dadurch gekennzeichnet, daß** durch ein gezieltes selektives Auftragen der Mischung der Silikonöle beide Endabschnitte des zylindrischen Bereiches unbeschichtet bleiben.

11. Verfahren nach einem der Ansprüche 7 bis 10, **dadurch gekennzeichnet, daß** das Auftragen der Mischung der Silikonöle unmittelbar nach dem Ausformen des Kunststoff-Hohlkörpers auf seine noch warme, eine Temperatur vorzugsweise über 40° C aufweisende Innenwandung erfolgt.

## Claims

1. Pharmaceutical packing device comprising an extended hollow plastic body (1) which is cylindrical over a portion or all of its axial length and is provided with a silicone lubricant layer (3) in this cylindrical region and is closeable by tight-fitting stoppers (14), **characterized in that** the lubricant layer (3) comprises a mixture of reactive silicone oil with nonreactive silicone oil, the fraction of nonreactive silicone oil in the mixture being in the range from 5% to 85% and the viscosity of the reactive silicone oil being in the range from 10 000 to 100 000 centistokes in the case of one-component systems and in the range from 200 to 5 000 centistokes in the case of multi-component systems and the viscosity of the nonreactive silicone oil being in the range from 350 to 20 000 centistokes.

2. Pharmaceutical packing device according to Claim 1, **characterized in that** the thickness of the lubricant layer (3) is in the range from 10 nm to 1 µm.

3. Pharmaceutical packing device according to Claim 1 or 2, **characterized in that** the cylindrical region of the hollow plastic body (1) is provided with the lubricant layer (3) over its full axial length.

4. Pharmaceutical packing device according to any of Claims 1 to 3, **characterized in that** one of the end sections of the cylindrical region of the hollow plastic body (1) is uncoated.

5. Pharmaceutical packing device according to any of Claims 1 to 3, **characterized in that** both end sections (A, B) of the cylindrical region of the hollow plastic body (1) are uncoated.

6. Pharmaceutical packing device according to any of Claims 1 to 5, **characterized in that** the plastic material of which the hollow plastic body (1) is made is a cycloolefin polymer or a copolymer thereof.

7. Method for making the pharmaceutical packing device comprising an extended hollow plastic body according to any of Claims 1 to 6, which comprises the steps:
- forming the hollow plastic body,
- providing a mixture of reactive silicone oil with nonreactive silicone oil,
- applying the mixture of the silicone oils to the interior surface of the hollow plastic body in the cylindrical region,
- cross-linking the mixed silicone oils in the applied layer, and
- further processing the hollow plastic body thus provided with a lubricant layer in accordance with its envisaged use.

8. Method according to Claim 7, **characterized in that** the applying of the mixture of silicone oils is effected selectively in relation to the axial extent of the cylindrical region.

9. Method according to Claim 8, **characterized in that** the mixture of silicone oils is applied selectively to leave one end section of the cylindrical region uncoated.

10. Method according to Claim 8, **characterized in that** the mixture of silicone oils is applied selectively to leave both end sections of the cylindrical region uncoated.

11. Method according to any of Claims 7 to 10, **characterized in that** the applying of the mixture of silicone oils is effected immediately after the forming of the hollow plastic body, onto its interior surface which is still hot and preferably has a temperature of above 40°C.

## Revendications

1. Moyen d'emballage pour produits pharmaceutiques, constitué d'un corps creux oblong en matière synthétique (1) qui est conformé au moins sur une partie de sa longueur axiale de façon cylindrique et est pourvu dans cette zone cylindrique d'une couche de glissement en silicone (3) et qui peut être fermé par des bouchons (14) placés de façon étanche, **caractérisé en ce que** la couche de glissement (3) est constituée d'un mélange d'une huile de silicone réactive et d'une huile de silicone non réactive, la proportion de l'huile de silicone non réactive dans le mélange se trouvant dans la plage de 5% à 85% et la viscosité de l'huile de silicone réactive se trouvant dans le cas de systèmes à un composant dans la plage de 10 000 à 100 000 centistokes, dans le cas de systèmes à plusieurs composants dans la plage de 200 à 5 000 centistokes, et la viscosité de l'huile de silicone non réactive se trouvant dans la plage de 350 à 20 000 centistokes.

2. Moyen d'emballage pour produits pharmaceutiques selon la revendication 1, **caractérisé en ce que** l'épaisseur de la couche de glissement (3) se situe dans la plage de 10 nm à 1 µm.

3. Moyen d'emballage pour produits pharmaceutiques selon la revendication 1 ou 2, **caractérisé en ce que** la zone cylindrique du corps creux en matière synthétique (1) est pourvue sur toute sa longueur axiale de la couche de glissement (3).

4. Moyen d'emballage pour produits pharmaceutiques selon l'une quelconque des revendications 1 à 3, **caractérisé en ce que** l'une des parties d'extrémité de la zone cylindrique du corps creux en matière synthétique (1) est non revêtue.

5. Moyen d'emballage pour produits pharmaceutiques selon l'une quelconque des revendications 1 à 3, **caractérisé en ce que** les deux parties d'extrémité (A, B) de la zone cylindrique du corps creux en matière synthétique (1) sont non revêtues.

6. Moyen d'emballage pour produits pharmaceutiques selon l'une quelconque des revendications 1 à 5, **caractérisé en ce que** la matière synthétique en laquelle le corps creux en matière synthétique (1) est fabriqué, est un polymère de cyclooléfine ou copolymère de cyclooléfine.

7. Procédé de fabrication du moyen d'emballage pour produits pharmaceutiques constitué d'un corps creux allongé en matière synthétique, selon l'une quelconque des revendications 1 à 6 et comportant les étapes de
- formage du corps creux en matière synthétique
- préparation d'un mélange d'une huile de silicone réactive et d'une huile de silicone non réactive
- application du mélange des huiles de silicone sur la paroi intérieure du corps creux en matière synthétique dans la zone cylindrique
- réticulation des huiles de silicones mélangées dans la couche appliquée et
- transformation ultérieure du corps creux en matière synthétique ainsi pourvu d'une couche de glissement, conformément à son utilisation prévue.

8. Procédé selon la revendication 7, **caractérisé en ce que** l'application du mélange des huiles de silicone s'effectue de façon ciblée par rapport à l'étendue axiale de la zone cylindrique.

9. Procédé selon la revendication 8, **caractérisé en ce qu'**une partie d'extrémité de la zone cylindrique reste non revêtue par une application sélective ciblée du mélange des huiles de silicone.

10. Procédé selon la revendication 8, **caractérisé en ce que** les deux parties d'extrémité de la zone cylindrique restent non revêtues par une application sélective ciblée du mélange des huiles de silicone.

11. Procédé selon l'une quelconque des revendications 7 à 10, **caractérisé en ce que** l'application du mélange des huiles de silicone s'effectue immédiatement après le formage du corps creux en matière synthétique sur sa paroi interne encore chaude, présentant une température de préférence supérieure à 40°C.
